# EUROPEAN PATENT APPLICATION

(11) **EP 2 589 357 A1**
(43) Date of publication of application: **08.05.2013**
(21) Application number: 11801015.6
(22) Date of filing: 27.06.2011
(51) Int. Cl.: A61F 13/15, A61F 13/53, A61F 13/534

(54) **THIN ABSORBENT ARTICLE**

(30) Priority: 30.06.2010 JP 2010150557
(71) Applicant: Unicharm Corporation, Ehime 799-0111 (JP)
(72) Inventor: NAKASHITA, Masashi, Kanonji-shi Kagawa 769-1602 (JP); MITSUNO, Satoshi, Kanonji-shi Kagawa 769-1602 (JP)
(74) Representative: Peter, Julian
(86) International application number: PCT/JP2011/065239
(87) International publication number: WO 2012/002553

(57) **Abstract**

Disclosed is an absorbent article containing an absorbent body which is thin before absorption, and even after absorption does not inhibit super absorbent polymer (SAP) swelling and wherein deformation such as twisting is unlikely to occur. The disclosed absorbent article comprises a liquid-permeable front surface sheet, a liquid impermeable rear surface sheet, and an absorbent body which is located between the two sheets. The absorbent body is formed from a super absorbent polymer containing layer and a non-woven fabric layer, with the non-woven fabric layer having regions wherein the between-fibre void ratio is relatively large and regions wherein the between-fibre void ratio is relatively small. The tip sections of the fibres forming the non-woven fabric layer are greater in number in the regions wherein the between-fibre void ratio is relatively large than the regions wherein the between-fibre void ratio is relatively small, and the non-woven fabric layer has expansibility.

## Description

### TECHNICAL FIELD

The present invention relates to a thin absorbent article such as a sanitary napkin or disposable diaper. More particularly, the present invention relates to an absorbent article that is thin prior to absorption, does not inhibit swelling of a superabsorbent polymer (SAP), and is resistant to the occurrence of deformation such as twisting.

### BACKGROUND ART

An absorbent body obtained by wrapping a mixture of short fibers and superabsorbent polymer particles with a wrapping sheet such as tissue is known as an absorbent body used in absorbent articles such as sanitary napkins or disposable diapers. For example, Japanese Unexamined Patent Publication No. 2008-125603 discloses an absorbent body having a superior liquid absorption rate and flexibility and an absorbent article provided with that absorbent body, the absorbent body is provided with at least one core layer consisting mainly of a web and absorbent polymer particles contained therein, the web is formed from crimped short fibers other than pulp fibers, the short fibers are not mutually fused, and the core layer does not substantially contain fibers other than the short fibers. Japanese Unexamined Patent Publication No. 2008-125603 also discloses a sheet-like absorbent body for enhancing spot absorbency of a liquid or improving shape retention of a web comprising interposing and anchoring a web between a pair of sheet materials, which is obtained by superimposing or covering one or a plurality of sheet materials such as paper or non-woven fabric, and joining or heat-fusing the web and the sheet materials with an adhesive applied to the sheet materials.

### DISCLOSURE OF THE INVENTION

### Problems to be Solved by the Invention

However, in the absorbent body disclosed in Japanese Unexamined Patent Publication No. 2008-125603, since the short fibers are not mutually fused and the core layer is substantially free of fibers other than the short fibers, there is susceptibility to the occurrence of deformation due to swelling of the absorbent polymer. In addition, although the superimposing of a layer of a non-woven fabric and the like above and/or below the web is disclosed in order to prevent deformation, in a structure in which a single web layer is simply interposed between a pair of non-woven fabric layers, twisting and separation end up occurring due to the amount of swelling of the absorbent polymer per layer becoming large, thereby resulting in a high risk of leakage due to a decrease in absorbency. In addition, if the amount of single fibers is increased in order to inhibit movement following swelling of the absorbent polymer, it becomes difficult to reduce weight and thickness. In addition, there is no mention made regarding anchoring of the absorbent polymer, and particularly in the case the absorbent polymer is not anchored, there is increased susceptibility to movement of the absorbent polymer and a high risk of the occurrence of discomfort when wearing and leakage due to a decrease in absorptive performance. Furthermore, in the case of anchoring the absorbent polymer by means of an adhesive, at least a portion of the surface of the absorbent polymer ends up being covered, again resulting in a high risk of leakage due to a decrease in absorptive performance.

### Means for Solving the Problems

The inventors of the present invention found that the aforementioned problems of the prior art are solved by using a non-woven fabric having regions where the inter-fiber void ratio is relatively large and regions where the inter-fiber void ratio is relatively small, wherein the ends of the fibers are more numerous in those regions where the inter-fiber void ratio is relatively large than in those regions where the inter-fiber void ratio is relative small, and the non-woven fabric has expansibility, thereby leading to completion of the present invention.

Namely, the present invention is an absorbent article having a liquid-permeable top sheet, a liquid-impermeable back sheet and an absorbent body positioned between both of these sheets, wherein the absorbent body is composed of a superabsorbent polymer-containing layer and a non-woven fabric layer, the non-woven fabric layer has regions where inter-fiber void ratio is relatively large and regions where inter-fiber void ratio is relatively small, the ends of the fibers that compose the non-woven fabric layer are more numerous in those regions where the inter-fiber void ratio is relatively large than in those regions where the inter-fiber void ratio is relatively small, and the non-woven fabric layer has expansibility.

The elasticity of the non-woven fabric layer preferably differs according to the direction.

The non-woven fabric preferably has elasticity of 1.4 times or more in at least one direction.

Regions where inter-fiber void ratio is relatively large and regions where inter-fiber void ratio is relatively small are preferably alternately formed continuously in the non-woven fabric layer, and the elasticity of the non-woven fabric layer is preferably greater in the direction perpendicular to the direction in which those regions where inter-fiber void ratio is relatively small extend than the direction in which those regions where inter-fiber void ratio is relatively small extend.

Elasticity in the direction perpendicular to the direction in which those regions where inter-fiber void ratio is relatively small extend is preferably 1.4 times or more, and elasticity in the direction in which those regions where inter-fiber void ratio is relatively small is less than 1.35 times.

The ends of the fibers are preferably fractured ends.

The non-woven fabric layer preferably has a density of 0.05 g/cm³ or less.

The non-woven fabric layer is preferably composed of a non-woven fabric processed with biting gear rollers.

The non-woven fabric layer is preferably composed of an air-through non-woven fabric processed with biting gear rollers having a tooth pitch of 2 mm to 8 mm and a tooth depth of 3 mm to 6 mm.

The absorbent body is preferably composed of at least one layer of a superabsorbent polymer-containing layer and at least two layers of a non-woven fabric layer, and the superabsorbent polymer-containing layer is preferably positioned between the non-woven fabric layers.

The superabsorbent polymer-containing layer preferably further contains moisture absorbent fibers.

At least a portion of fibers that compose the non-woven fabric layer and moisture absorbent fibers that compose the superabsorbent polymer-containing layer are preferably bonded at the interface between the non-woven fabric layers and the superabsorbent polymer-containing layer.

The absorbent body is preferably molded by applying water or steam.

The superabsorbent polymer-containing layer is preferably divided into a plurality of parts in a machine direction.

In addition, the present invention is an absorbent article having a liquid-permeable top sheet, a liquid-impermeable back sheet, an absorbent body positioned between both of these sheets, and a wrapping sheet that surrounds the absorbent body, wherein the absorbent body is composed of a superabsorbent polymer-containing layer and a non-woven fabric layer, the non-woven fabric layer has regions where inter-fiber void ratio is relatively large and regions where inter-fiber void ratio is relatively small, the ends of the fibers that compose the non-woven fabric layer are more numerous in those regions where the inter-fiber void ratio is relatively large than in those regions where the inter-fiber void ratio is relatively small, the non-woven fabric layer has elasticity of 1.4 times or more in at least one direction, and the density of the non-woven fabric layer is 0.05 g/cm³ or less.

### Effects of the Invention

Since absorbent article of the present invention has an absorbent body composed of a superabsorbent polymer-containing layer and a non-woven fabric layer, the non-woven fabric layer has regions where inter-fiber void ratio is relatively large and regions where inter-fiber void ratio is relatively small, the ends of the fibers that compose the non-woven fabric layer are more numerous in those regions where the inter-fiber void ratio is relatively large than in those regions where the inter-fiber void ratio is relatively small, and the non-woven fabric layer has expansibility, the non-woven fabric layer is able to match swelling of the superabsorbent polymer-containing layer, does not inhibit swelling of the superabsorbent polymer-containing layer, and is able to maximize effective utilization of the absorption capacity of the superabsorbent polymer-containing layer. In addition, as a result of having regions where inter-fiber void ratio is relatively large and regions where inter-fiber void ratio is relatively small, the ends of the fibers that compose the non-woven fabric layer being more numerous in those regions where the inter-fiber void ratio is relatively large than in those regions where the inter-fiber void ratio is relatively small, and the non-woven fabric layer having expansibility, the absorbent article of the present invention has superior moisture absorption ability and demonstrates little movement or deformation while worn. Thus, the absorbent article of the present invention, despite being thin prior to absorbing liquid, has adequate liquid absorption capacity and causes little discomfort when worn.

### BRIEF DESCRIPTION OF THE DRAWINGS

FIG. 1 is an overhead view of an embodiment of the absorbent article of the present invention.
FIG. 2 is a cross-sectional view taken along line X-X' of FIG. 1.
FIG. 3 is a drawing showing an example of biting gear rollers.
FIG. 4 is a cross-sectional view of an example of an absorbent body having a five-layer structure.
FIG. 5 is a cross-sectional view of an absorbent article of Example 1 of the present invention.
FIG. 6 is an overhead view of an embodiment of an absorbent body in which a superabsorbent polymer-containing layer is divided into a plurality of parts in a machine direction.
FIG. 7 is a cross-sectional view taken along line Y-Y' of FIG. 6.
FIG. 8 is a cross-sectional view of another embodiment of an absorbent body in which a superabsorbent polymer-containing layer is divided into a plurality of parts in a machine direction.
FIG. 9 is an overhead photograph of a non-woven fabric for use as a non-woven fabric used in Example 1 of the present invention.
FIG. 10 is a photomicrograph of a region where inter-fabric void ratio is relatively large in a non-woven fabric for use as a non-woven fabric used in Example 1 of the present invention.
FIG. 11 is a photomicrograph of a region where inter-fabric void ratio is relatively small in a non-woven fabric for use as a non-woven fabric used in Example 1 of the present invention.

### BEST MODE FOR CARRYING OUT THE INVENTION

Although the following provides an explanation of the present invention using the drawings, the present invention is not limited to that indicated in the drawings.

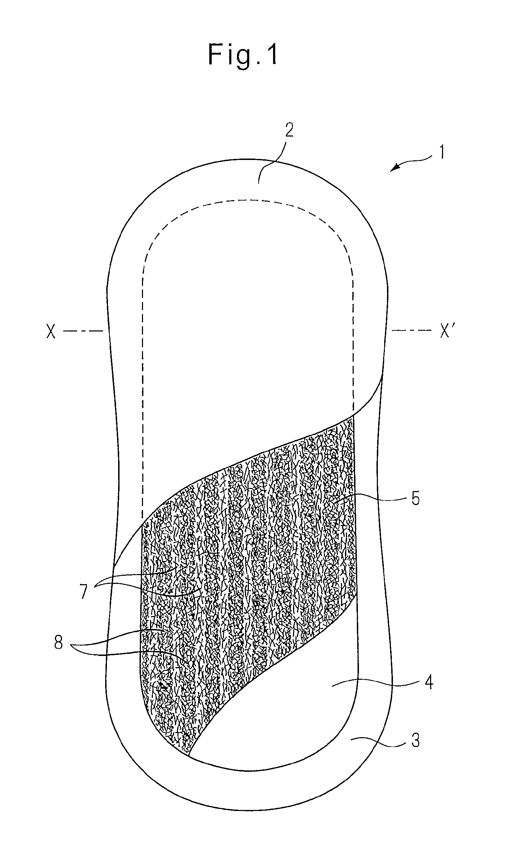

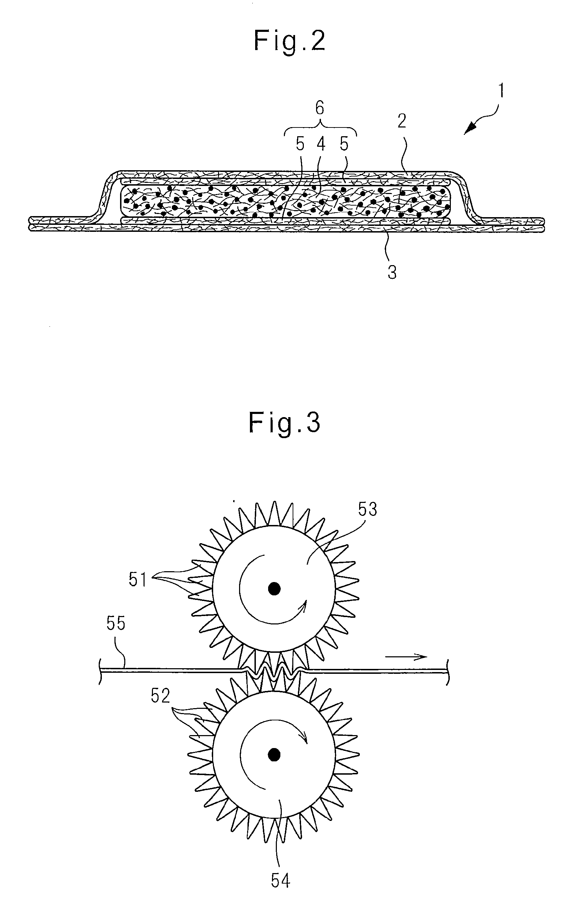

FIG. 1 is an overhead view of an embodiment of the absorbent article of the present invention, and a portion of each sheet or layer has been cut away to as to be able to partially visualize the interior. FIG. 2 is a cross-sectional view taken along line X-X' in FIG. 1.

An absorbent article 1 of the present invention has a liquid-permeable top sheet 2, a liquid-impermeable back sheet 3, and an absorbent body 6 positioned between both of these sheets, the absorbent body 6 is composed of a superabsorbent polymer-containing layer 4 and a non-woven fabric layer 5, the non-woven fabric layer 5 has regions 7 where inter-fiber void ratio is relatively large and regions 8 where inter-fiber void ratio is relatively small, the ends of the fibers that compose the non-woven fabric layer are more numerous in those regions where inter-fiber void ratio is relatively large than in those regions where inter-fiber void ratio is relatively small, and the non-woven fabric layer has expansibility.

Although the absorbent body 6 is depicted in FIG. 2 as being composed of one layer of the superabsorbent polymer-containing layer 4 and two layers of the non-woven fabric layer 5, the number of each layer is not limited to that depicted here.

The non-woven fabric layer 5 has regions 7 where inter-fiber void ratio is relatively large, and regions 8 where inter-fiber void ratio is relatively small. The end of the fibers that compose the non-woven fabric layer 5 are more numerous in those regions where inter-fiber void ratio is large than in those regions where inter-fiber void ratio is relatively small. In addition, the non-woven fabric layer 5 has expansibility. The non-woven fabric layer 5 is preferably composed of a non-woven fabric that has been processed with biting gear rollers. The non-woven fabric layer 5 is preferably formed to have room for stretching so as to be able to match swelling of the superabsorbent polymer by fracturing a portion of the fibers that compose the non-woven fabric or untangling the fibers. The use of this non-woven fabric enables the non-woven fabric layer to match swelling of the superabsorbent polymer when the absorbent body has absorbed a liquid such as menstrual blood or urine. As a result, effective utilization of the absorption capacity of the superabsorbent polymer can be maximized without inhibiting swelling of the superabsorbent polymer by the non-woven fabric layer. In addition, this non-woven fabric has superior moisture absorption ability, and demonstrates little movement or deformation when worn in comparison with cellulose pulp and the like. In addition, since this non-woven fabric is able to hold the superabsorbent polymer in the region 7 where inter-fiber void ratio is relatively large, there is little movement of the superabsorbent polymer while wearing. Thus, an absorbent article can be fabricated, which despite being thin prior to absorbing liquid, has adequate liquid absorption capacity and causes little discomfort when worn, and can also be reduced in both thickness and weight.

Inter-fiber voids refer to those portions where fibers are not present when the non-woven fabric is viewed from overhead. Inter-fiber void ratio refers to the ratio of the area of those portions where fibers are not present to the total of the area of portions where fibers are present and the area of portions where fibers are not present within a certain area, and is expressed as a percentage.

Inter-fiber void ratio can be measured using the methods indicated below.
(1) The non-woven fabric is placed on a black pasteboard and a photomicrograph is taken of the non-woven fabric.
(2) An image of the microphotograph is scanned with a scanner.
(3) Image analysis software in the form of USB Digital Scale (Scalar Corp.) is booted and the image captured in (2) is called up.
(4) After setting the binarization setting to 0 to 150, the captured image is converted by static binarization.
(5) The range of the target measurement region is specified.
(6) The shape feature extraction command is selected, black is set for the target color in the count tab, the width and height of the extraction target are specified to 0 pixels or more, and the count button is pressed to extract void portions.
(7) Next, the total area is specified in the extraction volume tab, and the feature value button is pressed to enable measurement of the area of black portions within the specified range.
(8) Inter-fiber void ratio is then calculated according to the equation indicated below.
   Inter-fiber void ratio = area of black portions within
   specified range/area of specified range
(9) The results of measurements for at least 10 points made according to the procedure described above are then averaged, and the resulting average value is used for the value of inter-fiber void ratio.

Although there are no particular limitations on the absolute value of inter-fiber void ratio of the non-woven fabric layer 5 provided it has regions 7 where inter-fiber void ratio is relatively large and regions 8 where inter-fiber void ratio is relative small, the inter-fiber void ratio of the regions 7 where inter-fiber void ratio is relative large is preferably 25% to 45%, and the inter-fiber void ratio of the regions 8 where inter-fiber void ratio is relatively small is preferably 50% to 70%.

Expansibility refers to the property of the non-woven fabric layer stretching in a direction in which tensile force is applied thereto when tensile force is applied in that direction. The non-woven fabric layer 5 has expansibility in at least one direction. For example, although the non-woven fabric layer 5 has expansibility in the transverse direction, it does not have expansibility in the longitudinal direction. Elasticity of the non-woven fabric 5 is preferably 1.4 times or more, more preferably 1.5 times or more, and even more preferably 1.6 times or more in the direct of greatest expansibility. Although there are no particular limitations on the upper limit of elasticity of the non-woven fabric 5, elasticity of the non-woven fabric 5 is preferably 2.5 times or less. Elasticity refers to the value obtained by dividing the length when stretched at a force or 1.0 N per 25 mm of width by the initial length. The method used to measure elasticity will be subsequently described. If elasticity is too small, the non-woven fabric layer 5 is unable to match swelling of the superabsorbent polymer when the superabsorbent polymer-containing layer has absorbed a liquid, while conversely if elasticity is too large, inter-fiber void ratio becomes excessively large, making it difficult to retain the superabsorbent polymer. The elasticity of the non-woven fabric layer 5 may differ according to direction. For example, in the case the non-woven fabric layer 5 is composed of a non-woven fabric that has been processed by biting gear rollers, elasticity in the direction in which the non-woven fabric has been stretched by the biting gear rollers is the largest, while elasticity in the direction perpendicular to that direction becomes the smallest. In the case of being processed by biting gear rollers in two directions consisting of the lengthwise direction and widthwise direction of the non-woven fabric, there are two directions in which elasticity is large. The non-woven fabric layer 5 preferably has continuously alternating regions where inter-fiber void ratio is relatively large and regions where inter-fiber void ratio is relatively small, and elasticity of the non-woven fabric layer is preferably larger in the direction perpendicular to the direction in which the regions where inter-fiber void is relatively small extend than in the direction in which the regions where inter-fiber void is relatively small extend. Elasticity in the direction perpendicular to the direction in which the regions where inter-fiber void is relatively small extend is preferably 1.4 times or more, while elasticity in the direction in which the regions where inter-fiber void is relatively small extend is less than 1.35 times. Furthermore, although there are no particular limitations on the direction when the non-woven fabric layer 5 is incorporated in an absorbent article, since the shape of the absorbent body is normally smaller in the widthwise direction than the lengthwise direction, and the ratio at which the non-woven fabric 5 expands is greater in the widthwise direction than in the lengthwise direction when the superabsorbent polymer has swelled, the direction in which elasticity of the non-woven fabric 5 is the largest is preferably made to coincide with the widthwise direction of the absorbent body.

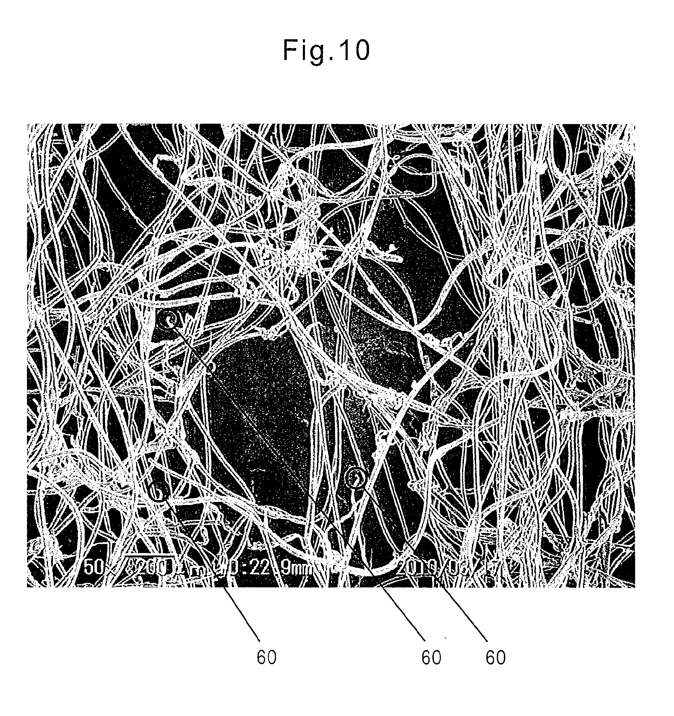

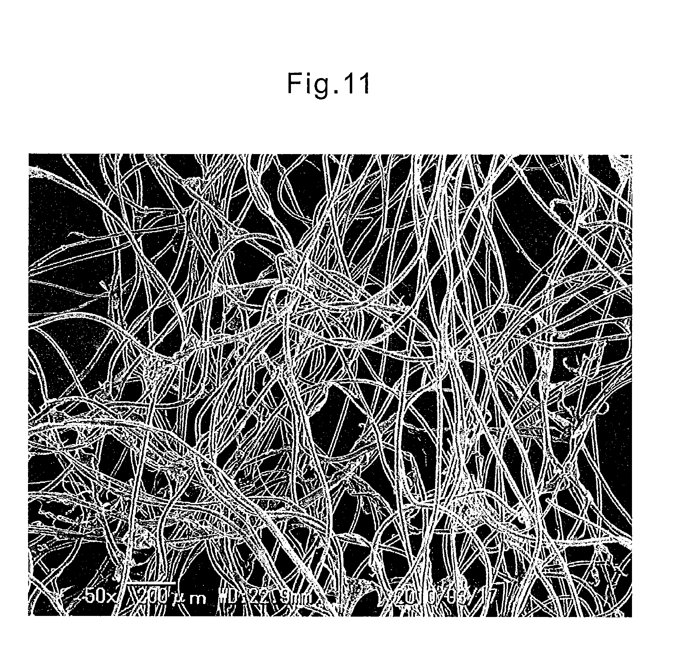

The ends of the fibers that compose the non-woven fabric layer 5 are more numerous in those regions where inter-fiber void ratio is relatively large than in those regions where inter-fiber void ratio is relatively small. The ends of the fibers are preferably fractured ends of the fibers. Fractured ends refer to fiber ends formed due to fracturing of fibers that have been stretched excessively when fibers that compose the non-woven fabric are stretched during processing of the non-woven fabric with biting gear rollers. FIG. 10 is a photomicrograph of a region where inter-fiber void ratio is relatively large, while FIG. 11 is a photomicrograph of a region where inter-fiber void ratio is relatively small. In FIG. 10, reference symbol 60 indicates fractured ends.

Density of the non-woven fabric layer 5 is preferably 0.05 g/cm³ or less and more preferably 0.02 g/cm³ to 0.05 g/cm³. If density is excessively large, decreases in liquid permeability and hardness end up occurring, while if density is excessively small, the superabsorbent polymer ends up falling out easily.

There are no particular limitations on the non-woven fabric that composes the non-woven fabric layer 5, examples include air-through non-woven fabric, spun lace non-woven fabric, spun bonded non-woven fabric, thermal bond non-woven fabric, melt blown non-woven fabric and needle punched non-woven fabric, and from the viewpoint of easily forming a bulky structure, an air-through non-woven fabric is preferable. In addition, there are also no particular limitations on the fibers that compose the non-woven fabric, examples include fibers of thermoplastic resins such as polyolefins, including polyethylene and polypropylene, or polyethylene terephthalate, and core-sheath type fibers and side-by-side composite fibers composed of a combination of these thermoplastic resins, and heat fusion fibers are used preferably. Examples of heat fusion fibers include polyester/polyethylene core-sheath type composite fibers. If heat fusion fibers are used for the fibers that compose the non-woven fabric layer 5, non-woven fabric layers 5 that are mutually adjacent in the direction of thickness can be easily bonded by heat fusion. In addition, as a result of fusing heat fusion fibers, heat fusion fibers with moisture absorbent fibers and heat fusion fibers with a superabsorbent polymer, excessive deformation of the absorbent body is prevented even when subjected to shear and wet conditions, thereby making it possible to inhibit decreases in absorptivity and reduce discomfort when worn. Fiber fineness is preferably 0.01 dtex to 20 dtex, and more preferably 1 dtex to 10 dtex. If fineness is excessively large, due to the decrease in the number of fibers, not only does it become difficult to retain the superabsorbent polymer between the fibers, but it also becomes difficult to inhibit swelling of the superabsorbent polymer, while conversely if fineness is excessively small, the fibers per se lose rigidity (stiffness) and it becomes difficult to maintain a framework structure composed of the fibers. In addition, fiber length is preferably 30 mm to 80 mm and more preferably 40 mm to 70 mm. If fiber length is excessively long, handling during manufacturing becomes difficult, while if fiber length is excessively short, there is a decrease in the number of locations of heat fusion, thereby making it difficult to form a framework structure composed of the fibers.

Although there are no particular limitations on the basis weight of the non-woven fabric layer 5, it is preferably 10 g/m² to 50 g/m² and more preferably 15 g/m² to 40 g/m². If the basis weight of the non-woven fabric layer 5 is excessively large, it becomes difficult to match the swelling of the absorbent body (superabsorbent polymer), while if the basis weight is excessively small, the non-woven fabric layer 5 is susceptible to tearing.

Although there are no particular limitations on the pitch at which the regions 7 where inter-fiber void ratio is relatively large and the regions 8 where inter-fiber void ratio is relatively small appear in the widthwise direction, lengthwise direction or diagonal direction (namely, the gap between the center line of the adjacent regions 7 where inter-fiber void ratio is relatively large and the adjacent regions 8 where inter-fiber void ratio is relatively small), it is preferably 1 mm to 20 mm and more preferably 2 mm to 20 mm. If the pitch is excessively large, the amount of stretching of the non-woven fabric layer 5 decreases, thereby making it difficult to match the swelling of the absorbent body (superabsorbent polymer), while conversely if the pitch is excessively small, the strength of the non-woven fabric layer 5 ends up decreasing. Although there are no particular limitations on the width of the regions 7 where inter-fiber void ratio is relatively large, it is preferably 0.5 mm to 15 mm and more preferably 1.5 mm to 9 mm. If the width is excessively large, the superabsorbent polymer falls out easily, while conversely if the width is excessively small, the amount of stretching decreases and swelling of the absorbent body (superabsorbent polymer) is inhibited.

The non-woven fabric that composes the non-woven fabric layer 5 can be obtained by processing an original fabric having uniform inter-fiber void ratio with biting gear rollers. As shown in FIG. 3, biting gear roller processing refers to passing an original non-woven fabric 55 through the gap between a pair of gear rollers 53 and 54 that rotate while causing a plurality of teeth 51 and 52 formed around the outer periphery thereof to mesh together, thereby causing the teeth 51 and 52 to partially cut the fibers of the non-woven fabric 55, narrow and lengthen the fibers by plastic deformation, or untangle the fibers. Although a plurality of the teeth 51 and 52 are shown to be extending in the widthwise direction of the gear rollers 53 and 54 in FIG. 3, gear rollers may also be employed in which the plurality of teeth 51 and 52 extend in the circumferential direction, or gear rollers may be employed in which the plurality of teeth 51 and 52 extend in the diagonal direction. In the case of using gear rollers in which the plurality of teeth 51 and 52 extend in the widthwise direction, a non-woven fabric is obtained in which regions where inter-fiber void ratio is relatively large and regions where inter-fiber void ratio is relatively small are mutually formed in the lengthwise direction, while conversely in the case of using gear rollers in which the plurality of teeth 51 and 52 extend in the circumferential direction, a non-woven fabric is obtained in which regions where inter-fiber void ratio is relatively large and regions where inter-fiber void ratio is relatively small are mutually formed in the widthwise direction, and in the case of using gear rollers in which the plurality of teeth 51 and 52 extend in the diagonal direction, a non-woven fabric is obtained in which regions where inter-fiber void ratio is relatively large and regions where inter-fiber void ratio is relatively small are mutually formed in the diagonal direction.

Although there are no particular limitations on the diameter of the gear rollers 53 and 54 used, it is preferably 300 mm to 600 mm and more preferably 450 mm to 600 mm. The pitch of the teeth 51 and 52 of the gear rollers is preferably 2 mm to 8 mm and more preferably 2 mm to 6 mm. The depth of the teeth 51 and 52 of the gear rollers is preferably 3 mm to 6 mm and more preferably 4 mm to 6 mm. The circumferential speed of the gear rollers 53 and 54 is preferably 50 m/min to 300 m/min and more preferably 100 m/min to 200 m/min. Here, circumferential speed refers to the speed of the tips of the teeth 51 and 52.

The superabsorbent polymer-containing layer 4 contains a superabsorbent polymer. Although the superabsorbent polymer-containing layer 4 may be composed of only a superabsorbent polymer, it preferably contains moisture absorbent fibers in addition to the superabsorbent polymer. Moreover, the superabsorbent polymer-containing layer 4 may also contain a resin other than the superabsorbent polymer or fibers other than the moisture absorbent fibers within a range that does not impair the effects of the present invention.

The superabsorbent polymer (to also be referred to as "SAP") has a three-dimensional mesh structure consisting of a suitably crosslinking water-soluble polymer, and although the superabsorbent polymer absorbs several hundred times to several thousand times its weight in water, it is essentially water-insoluble, does not release the water once it has been absorbed even if subjected to a certain degree of pressure, and examples thereof include starch-based, acrylic acid-based and amino acid-based granular, fibrous and foamed polymers. The superabsorbent polymer-containing layer preferably contains two or more types of superabsorbent polymers having different absorptive performance, at least one type of superabsorbent polymer has a vortex absorption rate of within 20 seconds and an absorption volume at a load of 2.0 kPa of 25 g/g or more. Here, vortex absorption rate refers to the time required by 2 g of SAP to absorb 50 g of physiological saline as determined in compliance with the "Testing method for water absorption capacity of super absorbent polymers" described in JIS K7224, while the absorption volume at a load of 2.0 kPa refers to the amount of physiological saline absorbed in 1 hour per gram of SAP at 2.0 kPa. If a superabsorbent polymer having a vortex absorption rate of within 20 seconds and an absorption volume at a load of 2.0 kPa of 25 g/g or more (to be referred to as the "first superabsorbent polymer") is used, since a space (thickness) is formed as a result of instantaneously swelling and liquid permeability does not decrease after swelling, a liquid can be absorbed into an absorbent body without allowing liquid to accumulate on the surface, thereby making it possible to prevent leakage.

A superabsorbent polymer having a large absorption capacity (to be referred to as the "second superabsorbent polymer") is preferably used for the superabsorbent polymer used in combination with the first superabsorbent polymer. Since absorption volume is inadequate in the case of only using the first superabsorbent polymer, combining with the use of the second superabsorbent polymer makes it possible to suppress increases in the amount of the superabsorbent resin, thereby enabling the weight of the absorbent body to be reduced. The absorption mechanism resulting from the use of these two types of superabsorbent polymers is as indicated below. (1) A liquid makes contact with the absorbent body and the fibers and superabsorbent polymer become wet. (2) Moisture absorption by the first superabsorbent polymer is accelerated first, and the volume of the superabsorbent polymer increases. Since the non-woven fabric layer and the moisture absorbent fibers have a degree of freedom between fibers, they do not inhibit the increase in volume of the superabsorbent polymer, the absorbent body is able to recover its thickness and a temporary liquid containment space is formed, thereby enabling spot absorption. (3) Liquid that continues to enter is contained in the previously formed temporary containment space, after which the liquid expands along the fibers, undergoes a relative decrease in absorption rate, and is absorbed by the second superabsorbent polymer having a large absorption volume. When these two types of superabsorbent polymers are used in combination, the weight ratio of the first superabsorbent polymer to the second superabsorbent polymer is preferably 5/95 to 50/50 and more preferably 10/90 to 30/70.

Commercially available products can be used for the superabsorbent polymers. Furthermore, although there are no particular limitations on the particle diameter of the superabsorbent polymers, the particle diameter of the first superabsorbent polymer is preferably 250 µm to 350 µm, and that of the second superabsorbent polymer is preferably 300 µm to 400 µm. The distance between moisture absorbent fibers prior to swelling of the superabsorbent polymers is preferably smaller than the particle diameters of the first superabsorbent polymer and the second superabsorbent polymer because this makes it easier to retain the superabsorbent polymers. Although there are no particular limitations thereon, the bulk density of the superabsorbent polymers is such that the bulk density of the first superabsorbent polymer is preferably 0.2 g/cm³ to 0.5 g/cm³ while the bulk density of the second superabsorbent polymer is preferably 0.5 g/cm³ to 1.0 g/cm³, and the bulk density of the first superabsorbent polymer is more preferably 0.3 g/cm³ to 0.4 g/cm³ while that of the second superabsorbent polymer is more preferably 0.6 g/cm³ to 0.8 g/cm³. Furthermore, bulk density refers to the value obtained by dividing the weight of a powder by the volume occupied thereby, and can be determined by filling a powder into a container of known volume and measuring the weight thereof.

Examples of the moisture absorbent fibers include pulp flap, chemical pulp, cellulose fibers, rayon and acetate fibers. Arranging a superabsorbent polymer within moisture absorbent fibers causes hydrogen bonds between the water absorbent fibers and between the water absorbent fibers and the superabsorbent polymer to be broken due to absorption of liquid, thereby enabling the superabsorbent polymer to freely swell, and since swelling of the superabsorbent polymer is not inhibited, the absorption volume of the superabsorbent polymer can be used effectively, thereby making it possible to reduce the weight of the absorbent body.

Acetate fibers are preferably used for the moisture absorbent fibers. Since acetate fibers cause hydrogen bonding between acetate fibers and between the acetate fibers and the superabsorbent polymer as a result of having hydroxyl groups, movement of the superabsorbent polymer is prevented and dry thickness (shape) can be maintained. In addition, since it becomes difficult for the fibers to retain liquid and liquid mobility into the superabsorbent polymer is enhanced as a result of partially substituting hydroxyl groups with acetic acid groups, the presence of residual liquid between fibers can be reduced, the backflow of liquid can be reduced, and moistness can be inhibited. Since the use of acetate fibers having a Y-shaped cross-section serves to increase specific surface area, contact area between fibers and between the fibers and the superabsorbent polymer increases, thereby making it possible for bonds to form easily, to prevent movement of the superabsorbent polymer, and prevent discomfort when worn. In addition, since capillary effects are able to act more easily, diffusion of liquid can be promoted. Arranging a superabsorbent polymer within acetate fibers causes hydrogen bonds between the fibers and between the fibers and the superabsorbent polymer to be broken as a result of absorbing liquid, and the superabsorbent resin is able to swell freely. Alternatively arranging a superabsorbent polymer-containing layer containing acetate fibers and a non-woven fabric layer facilitates movement of liquid into the superabsorbent polymer due to the presence of the superabsorbent polymer between the acetate fibers, while at the same time breaking the hydrogen bonds between fibers, thereby making it possible to match the swelling of the superabsorbent polymer and allow deformation of the fiber framework.

Although there are no particular limitations on fiber length of the moisture absorbent fibers, it is preferably 20 mm to 70 mm. If the fiber length is excessively long, the propagation of liquid along the fibers is inhibited, while conversely if the fiber length is excessively short, the fibers have difficulty in entangling and maintain their shape, thereby making it possible for the superabsorbent resin to easily fall out.

The superabsorbent polymer-containing layer is preferably divided into a plurality of parts in a machine direction.

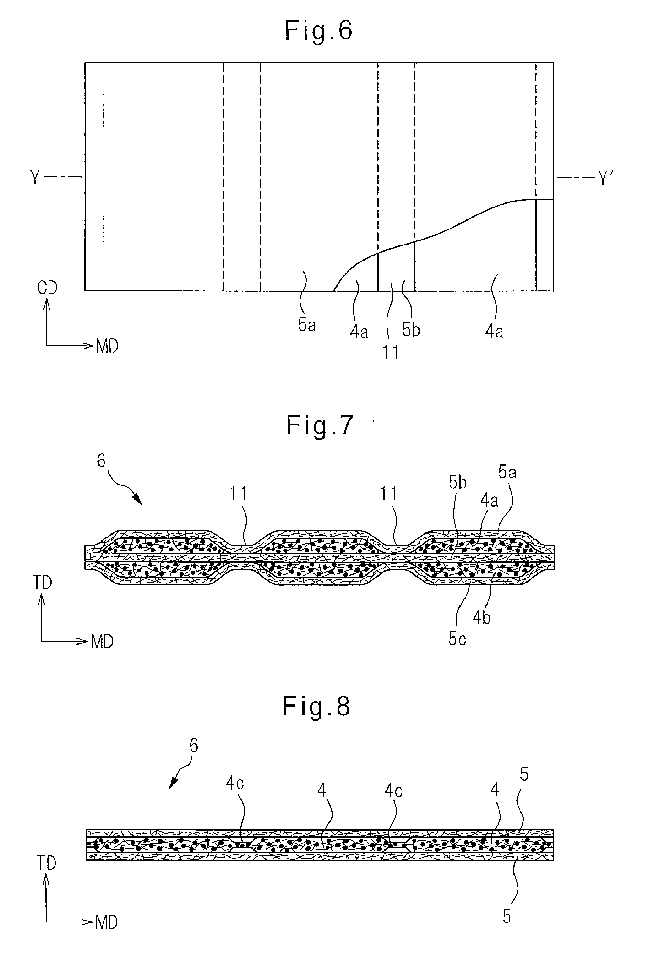

FIG. 6 is an overhead view of an embodiment of an absorbent body in which a superabsorbent polymer-containing layer is divided into a plurality of parts in a machine direction, and a portion of the non-woven fabric layer has been cut away to as to be able to partially visualize the interior. FIG. 7 is a cross-sectional view taken along line Y-Y' of FIG. 6. In the drawings, MD indicates the machine direction, CD indicates the cross-machine direction, and TD indicates the thickness direction. A superabsorbent polymer-containing layer 4a is divided into three parts in the machine direction. As a result of being divided in this manner, a region 11 where the superabsorbent polymer-containing layer is not present serves as a bending starting point that enables flexible deformation of the superabsorbent polymer-containing layer. In the embodiment shown in FIG. 7, non-woven fabric layers 5 (non-woven fabric layer 5a and non-woven fabric layer 5b) adjacent to each other in the thickness direction are joined in the region 11 between the superabsorbent polymer-containing layer 4a and a superabsorbent polymer-containing layer 4b. These joined portions serve as bending starting points while also inhibiting movement of the superabsorbent polymer-containing layers and reducing discomfort.

FIG. 8 is a cross-sectional view of another embodiment of an absorbent body in which a superabsorbent polymer-containing layer is divided into a plurality of parts in the machine direction. As shown in FIG. 8, the superabsorbent polymer-containing layers 4 adjacent to each other in the machine direction are not completely separated, but rather the superabsorbent polymer-containing layers 4 adjacent to each other in the machine direction may be connected by a portion 4c (low basis weight region) having a smaller basis weight than other portions. In such a case, the portion 4c having a small basis weight serves as a bending starting point, and enables flexible deformation of the absorbent article.

When fabricating an absorbent body in which superabsorbent polymer-containing layers are divided into a plurality of parts in the machine direction, a plurality of superabsorbent polymer-containing layers divided in advance may be arranged on a non-woven fabric while leaving gaps there between, or the superabsorbent polymer-containing layers may be arranged on the non-woven fabric followed by cutting only the superabsorbent polymer-containing layers.

The thickness of the superabsorbent polymer-containing layer 4 is preferably 1 mm to 20 mm and more preferably 2 mm to 10 mm. Although there are no particular limitations on the basis weight of the superabsorbent polymer containing-layer 4, it is preferably 10 g/m² to 1000 g/m² and more preferably 100 g/m² to 500 g/m². If the thickness and basis weight of the superabsorbent polymer-containing layer 4 are excessively large, it becomes difficult to anchor the superabsorbent polymer and it becomes difficult for the non-woven fabric layer 5 to match deformation of the absorbent body due to the large increase in swelling volume, while conversely if the thickness and basis weight are excessively small, absorption capacity ends up becoming small.

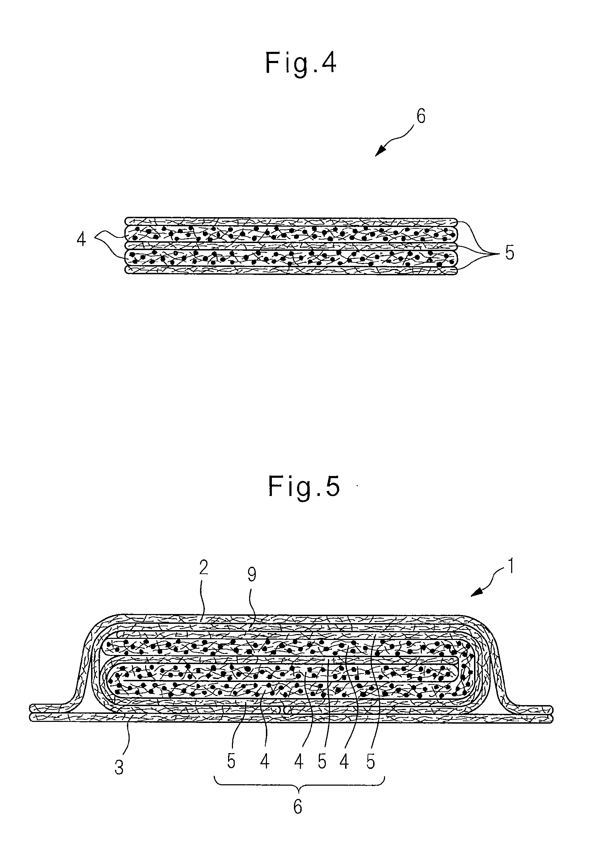

The absorbent body is preferably composed of at least one layer of the superabsorbent polymer-containing layer 4 and at least two layers of the non-woven fabric layer 5, and the superabsorbent polymer-containing layer 4 is preferably positioned between the non-woven fabric layers 5. Although FIG. 2 shows an absorbent body having a three-layer structure in which one layer of the superabsorbent polymer-containing layer 4 is arranged between two layers of the non-woven fabric layer 5, the absorbent body may also have a five-layer structure composed of three layers of the non-woven fabric layer and two layers of the superabsorbent polymer-containing layer, may have a seven-layer structure composed of four layers of the non-woven fabric layer and three layers of the superabsorbent polymer-containing layer, or may have another multilayer structure. FIG. 4 is a cross-sectional view of an example of the absorbent body 6 having a five-layer structure composed of three layers of the non-woven fabric layer 5 and two layers of the superabsorbent polymer-containing layer 4. As a result of alternately laminating the non-woven fabric layers 5 and the superabsorbent polymer-containing layers 4, liquid flow paths are formed by the formation of interfaces that facilitate the movement of liquid to the superabsorbent polymer. In addition, as a result of separately arranging those layers where the superabsorbent resin is allowed to swell (superabsorbent polymer-containing layer 4) and those layers that maintain shape (non-woven fabric layer 5), both absorption and shape retention are realized, thereby making it possible to reduce thickness while preventing leakage. In addition, in the case of a multilayer structure containing two or more layers of the superabsorbent polymer-containing layer 4, in comparison with the case of a single layer of the superabsorbent polymer-containing layer 4, since the amount of superabsorbent polymer per layer becomes less, the non-woven fabric layer follows the superabsorbent polymer within the layers even after the superabsorbent polymer has swollen, thereby making it possible to inhibit movement of the superabsorbent resin and resist the occurrence of deformation while wearing. If the superabsorbent polymer-containing layer 4 consists of two layers or more, although each superabsorbent polymer-containing layer 4 becomes thin, this thinness makes it easy for liquid to reach the superabsorbent polymer. In addition, since a thin superabsorbent polymer-containing layer results in the absorbent fibers becoming sparse, the superabsorbent polymer can be dispersed between the absorbent fibers.

In addition, as shown in FIG. 5, the absorbent body 6 may be folded three ways so that the non-woven layer 5 is located on the outside of a laminate of the superabsorbent polymer-containing layer 4 and the non-woven fabric layer 5. An absorbent body having such a structure has the advantage of facilitating positioning during production. In addition, folding a plurality of the non-woven fabric layers 5 so as to superimpose the superabsorbent polymer-containing layer 4 make it difficult for leakage from the superabsorbent polymer and the like to occur.

In addition, an absorbent body having a multi-layer structure may also be fabricated by laminating the superabsorbent polymer-containing layer on the non-woven fabric layer 5 and rolling it up therein. According to this fabrication method, a layer structure can be formed easily, thereby making it possible to simplify the fabrication process.

At least a portion of fibers that compose the non-woven fabric layer 5 and the moisture absorbent fibers that compose that superabsorbent polymer-containing layer 4 are preferably bonded at the interface between the non-woven fabric layer 5 and the superabsorbent polymer-containing layer 4. This bonding can be formed by, for example, spraying steam onto a laminate of the non-woven fabric layer 5 and the superabsorbent polymer-containing layer 4 and then heat fusing the fibers that compose the non-woven fabric layer 5 and the moisture absorbent fibers that compose the superabsorbent polymer-containing layer 4. As a result of bonding at least a portion of the fibers that compose the non-woven fabric layer 5 and the moisture absorbent fibers that compose that superabsorbent polymer-containing layer 4, the thickness of the absorbent article is kept thin and resistant to deformation in the worn state prior to voiding. Bonding at least a portion of the fibers that compose the non-woven fabric layer 5 and the moisture absorbent fibers that compose that superabsorbent polymer-containing layer 4 is effective in preventing further deformation even after the superabsorbent polymer has absorbed liquid and swollen.

The absorbent body 6 is preferably molded by applying water or steam. The application of water or steam can be carried out by, for example, spraying with water or steam. The application of water or steam enables the moisture absorbent fibers as well as the moisture absorbent fibers and the superabsorbent polymer to be anchored by hydrogen bonding. As a result, the absorbent body can be molded to have a thin shape. Hydrogen bonding between the moisture absorbent fibers and between the moisture absorbent fibers and the superabsorbent polymer serves to maintain a thin shape when the absorbent article is worn prior to voiding and makes the absorbent article resistant to deformation.

Since spraying of steam can be carried out simultaneously to the application of moisture, heat or force, hydrogen bonds are formed in the moisture absorbent fibers, heat fusion occurs in the non-woven fabric layer composed of a thermoplastic resin, and the fibers as well as the fibers and the superabsorbent polymer are anchored, and since the fibers are pushed apart by the force of the spraying, it becomes physically easier for the fibers per se as well as the fibers and the superabsorbent polymer to become entangled, thereby achieving maintenance of the thin shape and prevention of movement of the superabsorbent polymer, while also making it possible to reduce discomfort when worn. Furthermore, suction may also be used when spraying steam.

The thickness of the absorbent body is preferably 0.5 mm to 10 mm and more preferably 1 mm to 5 mm. If the absorbent body is excessively thick, since the thickness of the entire absorbent article increases, discomfort ends up occurring when wearing, while conversely if the absorbent body is excessively thin, it becomes difficult to secure adequate absorption capacity and the strength of the absorbent body decreases.

In the absorbent article of the present invention, the absorbent body may be further wrapped with a wrapping sheet 9 as shown in FIG. 5. As a result of wrapping with the wrapping sheet 9, the superabsorbent polymer can be prevented from coming out of the superabsorbent polymer-containing layer 4. Examples of materials used to compose the wrapping sheet 9 include tissue as well as woven or non-woven fabrics formed from a cellulose material such as cotton, recycled cellulose materials such as rayon or fibril rayon, semi-synthetic cellulose materials such as acetate or triacetate, fibrous polymers or thermoplastic hydrophobic chemical fibers. In particular, spunbond/melt blown/spunbond (SMS) non-woven fabric is preferable.

The absorbent body 6 can be fabricated in the manner described below. A superabsorbent polymer-containing layer is placed on a non-woven fabric to be used for the non-woven fabric layer, and a non-woven fabric is then placed thereon to fabricate an absorbent body having a three-layer structure. When fabricating an absorbent body having a five-layer structure, a superabsorbent polymer-containing layer is again placed on the absorbent body having a three-layer structure followed by placing a non-woven fabric thereon. An absorbent body having a greater number of layers can be fabricated by repeating layering of the superabsorbent polymer-containing layer and non-woven fabric. In the case the superabsorbent polymer-containing layer contains moisture absorbent fibers, although a mixture obtained by mixing the superabsorbent polymer into the moisture absorbent fibers may be placed on the non-woven fabric, opened moisture absorbent fibers may be arranged on the non-woven fabric followed by sprinkling the superabsorbent polymer thereon. At that time, water may be sprayed on the moisture absorbent fibers prior to sprinkling the superabsorbent polymer. Spraying with water makes it possible to inhibit movement of the sprinkled superabsorbent polymer and prevent the superabsorbent polymer from dissipating. After having placed the moisture absorbent fibers and superabsorbent polymer on the non-woven fabric, the laminate may be folded three ways so that the non-woven fabric layer 5 is on the outside to fabricate an absorbent having a five-layer structure. The laminate of the non-woven fabric layers and the superabsorbent polymer-containing layers may also be sprayed with steam. Spraying with steam serves to anchor each material that composes the laminate and enables the laminate to be formed to have a thin shape. The laminate may be pressed to form a thin shape instead of spraying with steam. The laminate may further be wrapped with a wrapping sheet such as tissue of SMS non-woven fabric.

The liquid-permeable top sheet 2 that composes the absorbent article 1 of the present invention has a function that allows liquid body waste such as menstrual blood or urine to pass through to the absorbent body 6 provided in a lower layer thereof, while also serving to hold the absorbent body 6 by interposing the absorbent body 6 between the top sheet 2 and the liquid-impermeable back sheet 3. All of a portion of the liquid-permeable top sheet 2 is liquid-permeable, and the liquid-permeable area is formed with a resin film in which a large number of liquid through holes have been formed, a net-like sheet having a large number of openings or liquid-permeable non-woven or woven fabric. A resin film or net-like sheet formed from polypropylene (PP), polyethylene (PE) or polyethylene terephthalate (PET) and the like can be used for the resin film of net-like sheet. In addition, a spunbond non-woven fabric formed from cellulose fibers such as rayon or synthetic resin fibers, or an air-through non-woven fabric formed from synthetic resin fibers, can be used for the non-woven fabric. A biodegradable, natural material such as polylactic acid, chitosan or polyalginic acid can also be used for the material. In addition, together with forming a large number of liquid through holes, the liquid-permeable top sheet 2 may be coated with a silicon-based or fluorine-based water-repellent oily agent to resist adhesion of liquid to the outside thereof.

Although there are no particular limitations on the basis weight of the liquid-permeable top sheet 2, it is preferably 10 g/m² to 50 g/m² and more preferably 20 g/m² to 40 g/m². If the basis weight of the liquid-permeable top sheet 2 is excessively small, adequate surface strength cannot be obtained resulting in the risk of tearing during use. In addition, if the basis weight is conversely excessively large, excessive stiffness occurs resulting in discomfort during use. Moreover, in the case of long-term use, liquid ends up being retained in the liquid-permeable top sheet and a sticky state is continued to be maintained, thereby resulting in an unpleasant sensation.

The liquid-impermeable back sheet 3 that composes the absorbent article 1 of the present invention has a function that prevents leakage of body fluids such as menstrual blood or urine absorbed by the absorbent body to the outside, and a material is used that is able to prevent leakage of such body fluids to the outside. In addition, by using a material that is impermeable to liquid but permeable to air, the sense of moistness when wearing can be reduced, thereby making it possible to reduce discomfort when worn. Examples of such materials include liquid-impermeable films composed mainly of polyethylene (PE) or polypropylene (PP) and the like, breathable film, and composite sheets obtained by laminating a liquid-impermeable film onto one side of a spunbond or other non-woven fabric. A hydrophobic non-woven fabric, water-impermeable plastic film or laminated sheet of a non-woven fabric and water-impermeable plastic film can be used preferably. In addition, an SMS non-woven fabric may also be used in which a highly water-resistant melt blown non-woven fabric is interposed between high-strength spunbond non-woven fabrics.

The absorbent article 1 can be fabricated by superimposing the liquid-permeable top sheet 2, the liquid-impermeable back sheet 3 and the absorbent body 6 using a commonly used method. More specifically, the absorbent body 6 is placed on the liquid-impermeable back sheet 3, and the liquid-permeable top sheet 2 is placed thereon followed by partially joining the liquid-permeable top sheet 2 and the liquid-impermeable back sheet 3 with an adhesive, heat fusion, or preferably heat embossing, and then cutting to desired dimensions and shape to fabricate the absorbent article 1.

The thickness of the absorbent article is preferably 1 mm to 20 mm and more preferably 2 mm to 15 mm. If the absorbent article is excessively thick, the feel when wearing the absorbent article becomes poor, while conversely if the absorbent article is excessively thin, it becomes difficult to secure adequate absorption capacity.

The absorbent article of the present invention is preferably used as a sanitary napkin or disposable diaper and the like. When using as a sanitary napkin or disposable diaper, the absorbent article is worn so that the side having the liquid-permeable top sheet contacts the skin of the wearer. There are no particular limitations on the shape of the absorbent article provided it has a shape that matches the shape of a woman's body or shorts, such as a rectangular shape, oval shape, hourglass shape or a shape provided with so-called wings to prevent slipping out of position with shorts in the case of using as a sanitary napkin, for example. The overall outer dimensions are preferably 100 mm to 500 mm and more preferably 150 mm to 350 mm in the lengthwise direction, and preferably 30 mm to 200 mm and more preferably 40 mm to 180 mm in the widthwise direction. Examples

### Example 1

An absorbent article having the layered structure shown in FIG. 5 was fabricated in the manner described below.

An air-through non-woven fabric (basis weight: 25 g/m²) composed of polyester/polyethylene composite fibers was used for the non-woven fabric of the non-woven fabric layer 5.

Bundles of acetate fibers were used as water absorbent fibers.

A superabsorbent polymer composed of an acrylic acid (acrylate) polymer having a vortex absorption rate of 3 seconds, absorption volume at a load of 2.0 kPa of 26 g/g and bulk density of 0.36 g/cm³ was used for the first superabsorbent polymer, and a superabsorbent polymer composed of an acrylic acid (acrylate) polymer having a vortex absorption rate of 30 seconds, absorption volume at a load of 2.0 kPa of 33 g/g and bulk density of 0.69 g/cm³ was used for the second superabsorbent polymer.

A spunbond/melt blown/spun bond non-woven fabric (SMS non-woven fabric) was used for the wrapping sheet 9.

An air-through non-woven fabric (basis weight: 25 g/m²) composed of polyester/polyethylene core-sheath composite fibers was used for the liquid-permeable top sheet 2.

A breathable film (basis weight: 18 g/m²) composed of polyethylene was used for the liquid-impermeable back sheet 3.

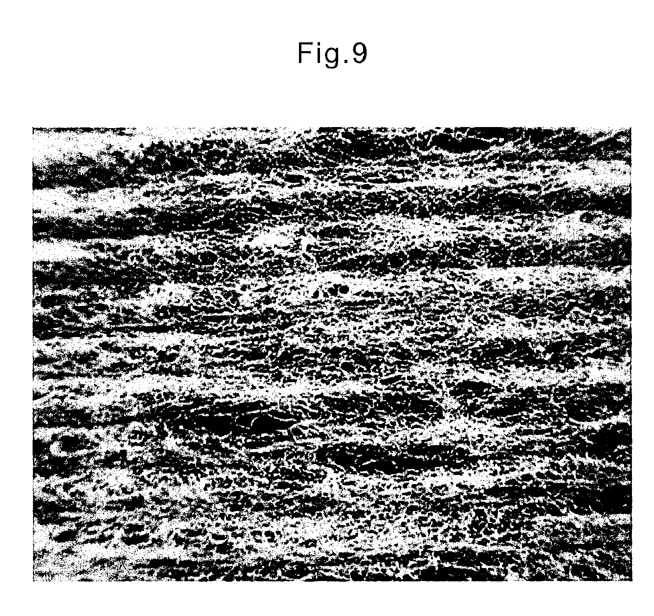

First, the air-through non-woven fabric for use in the non-woven fabric layer was passed between biting gear rollers (tooth pitch: 2.5 mm, processing speed: 10 m/min) in which teeth having a depth of 6 mm were extending in the circumferential direction to obtain a non-woven fabric (basis weight: 16 g/m²) in which regions where inter-fiber void ratio is relatively large and regions where inter-fiber void ratio is relatively small are alternately formed in the widthwise direction. A photograph of the resulting non-woven fabric subjected to biting gear roller processing is shown in FIG. 9. In FIG. 9, the vertical direction is the widthwise direction. The inter-fiber void ratio of regions where inter-fiber void ratio is relatively large was 60%, and the inter-fiber void ratio of regions where inter-fiber void ratio is relatively small was 38%. Elasticity of the non-woven fabric in the widthwise direction was 2.35. After cutting the non-woven fabric (1 g) subjected to biting gear roller processing to a length of 300 mm and width of 210 mm, 2 g of bundles of opened acetate fibers of the same size were cut to a length of 50 mm and arranged on the non-woven fabric over the width thereof (basis weight: 32 g/m²). Water was then sprayed onto the acetate fibers, and 1 g of the first superabsorbent polymer and 9 g of the second superabsorbent polymer were sprinkled over an area having a length of 270 mm and width of 210 mm (basis weight of first superabsorbent polymer: 18 g/m², basis weight of second superabsorbent polymer: 159 g/m²). A laminate of the non-woven fabric, acetate fibers and superabsorbent polymer was then followed three ways in the widthwise direction and molded to a width of 70 mm. Subsequently, steam was sprayed (pressure: 0.7 MPa, clearance: 2 mm) to fabricate an absorbent body. The resulting absorbent body had a weight of 1.41 g, thickness of 2.50 mm, basis weight of 744 g/m² and density of 0.30 g/cm³. The absorbent body was then wrapped in the SMS non-woven fabric and placed on a breathable film used for the liquid-impermeable back sheet, and an air-through non-woven fabric used for the liquid-permeable top sheet was superimposed thereon to fabricate an absorbent article.

### Example 2

An absorbent article was fabricated by fabricating an absorbent body in the same manner as Example 1 with the exception of changing the moisture absorbent fibers to a spun lace non-woven fabric (SL non-woven fabric) subjected processing with biting gear rollers having teeth having a depth of 6 mm extending in the circumferential direction, changing the gap clearance of the steam spraying nozzles to 2 mm, and changing the pressure to 0.5 MPa.

### Example 3

An absorbent article was fabricated by fabricating an absorbent body in the same manner as Example 2 with the exception of changing steam spraying to flat pressing (pressure: 20 kg/cm², duration: 10 seconds, clearance: 0 mm).

### Example 4

An absorbent article was fabricated by fabricating an absorbent body in the same manner as Example 3 with the exception of not using moisture absorbent fibers and changing the amount of air-through non-woven fabric used in the non-woven fabric layer processed with biting gear rollers to 3 g (basis weight: 144 g/m²).

### Example 5

An absorbent article was fabricated by fabricating an absorbent body in the same manner as Example 4 with the exception of changing the depth of the teeth of the biting gear rollers to 3 mm.

### Comparative Example 1

An absorbent article was fabricated by fabricating an absorbent body in the same manner as Example 1 with the exception of not carrying out biting gear roller processing.

The weight, thickness, absorption time and shape retention ratio (before absorption and after absorbing 160 mL) were evaluated for the absorbent articles fabricated in the examples and comparative example. The evaluation results are shown in Table 1.

**Table 1**

| | | | Example 1 | Example 2 | Example 3 | Example 4 | Example 5 | Comparative Example 1 |
|---|---|---|---|---|---|---|---|---|
| Non-woven fabric layer | Type | | TA non-woven fabric | TA non-woven fabric | TA non-woven fabric | TA non-woven fabric | TA non-woven fabric | TA non-woven fabric |
| | Gear roller processing | | Yes | Yes | Yes | Yes | Yes | No |
| | Biting depth | mm | 6 | 6 | 6 | 6 | 3 | - |
| | Elasticity | | 2.35 | 2.35 | 2.35 | 2.35 | 1.4 | 1.31 |
| Moisture absorbent fibers | Type | | Acetate fibers | SL non-woven fabric | SL non-woven fabric | - | - | Acetate fibers |
| | Gear roller processing | | | Yes | Yes | | | |
| | Biting depth | mm | | 6 | 6 | | | |
| | Elasticity | | | 1.78 | 1.78 | | | |
| Finished product | Weight | g | 17.3 | 17.2 | 17.2 | 17.0 | 16.4 | 17.3 |
| | Thickness | mm | 4.1 | 4.1 | 4.2 | 4.2 | 4.2 | 4.3 |
| Absorption time | 80 mL | s | 18 | 17 | 19 | 20 | 18 | 25 |
| | 160 mL | s | 20 | 21 | 21 | 20 | 20 | 32 |
| Shape retention ratio | Before absorption | % | 100 | 100 | 100 | 100 | 100 | 100 |
| | After absorbing 160 mL | % | 86 | 79 | 79 | 86 | 71 | 43 |

As shown in Table 1, the absorbent articles of the present invention (Examples 1 to 5) demonstrated shorter absorption times and higher shape retention ratios after absorption in comparison with Comparative Example 1.

Furthermore, the methods used to evaluate each of the evaluation parameters of Table 1 were as described below.

### [Weight]

Weight was measured using an electronic balance.

### [Thickness]

Thickness was measured using a thickness gauge (Peacock Corp., Model J-B, measuring surface: 50 mmφ, measuring pressure: 3 g/cm²). Thickness was measured by placing the location of the absorbent body were liquid was dropped thereon between the measuring stage and the measuring probe.

### [Absorption Time]

1) The absorbent body was arranged in a U-shaped apparatus, and a prescribed amount of liquid dropped at a rate of 8 mL/s using a dropping funnel was placed in the apparatus using 80 mL of artificial urine.
2) The amount of time until liquid overflowing to the side of the absorbent body was absorbed into the absorbent body (state in which the liquid does not flow out even if tilted slightly) was recorded.
3) 40 ml of liquid were dropped at 3 minute intervals, and time was measured until this was repeated for 200 mL.

### [Shape Retention Ratio (Before Absorption and after Absorbing 160 mL)]

Shape retention ratio was determined according to the following procedure for the absorbent articles before absorption and after absorbing 160 mL.
(1) The initial width of the absorbent body in the absorbent article was measured.
(2) The ends of a 50 mm region located in the center in the lengthwise direction of the absorbent body were anchored in the absorbent article.
(3) Both ends were brought closer to each other until the width of the absorbent body became 30 mm followed by anchoring for 30 seconds.
(4) Anchoring was then discontinued and a 3.5 kg weight (10 cm x 10 cm) was placed on the absorbent body for 30 seconds.
(5) The weight was removed and the procedures of (3) and (4) were repeated 5 times.
(6) The width of the narrowest portion of the absorbent body ("width after deformation") was measured 1 minute after having removed the weight for the fifth time.
(7) The "width after deformation" was divided by the "absorbent body initial width" to determine the shape retention ratio as a percentage.

### Example 6

Non-woven fabrics having different degrees of fiber cutting were fabricated by passing non-woven fabrics between biting gear rollers having various different tooth depths, followed by evaluating their thickness, density, elasticity and moisture absorption multiple. Air-through non-woven fabric and spun lace non-woven fabric having a length of 100 mm and width of 100 mm were used for the original non-woven fabrics. The evaluation results are shown in Table 2.

**Table 2**

| | | Air-through non-woven fabric | | | | | Spun lace non-woven fabric | | | |
|---|---|---|---|---|---|---|---|---|---|---|
| Tooth depth | mm | 0 | 3 | 4 | 5 | 6 | 0 | 4 | 5 | 6 |
| Thickness | mm | 1.46 | 8.27 | 10.69 | 10.57 | 14.73 | 0.75 | 6.55 | 9.76 | 12.60 |
| Density | g/cm³ | 0.074 | 0.014 | 0.009 | 0.008 | 0.006 | 0.142 | 0.025 | 0.016 | 0.011 |
| Elasticity | times | 1.31 | 1.55 | 1.67 | 2.27 | 2.35 | 1.20 | 1.41 | 1.58 | 1.78 |
| Moisture absorption multiple | g/g | 21 | 45 | 59 | 65 | 62 | 14 | 33 | 36 | 40 |

As shown in Table 2, the moisture absorption multiple was observed to improve the greater the gear biting depth. In addition, the moisture absorption multiple improved considerably at elasticity of 1.4 times or more.

Incidentally, the inter-fiber void ratio of those regions of air-through non-woven fabric subjected to biting gear roller processing at tooth depth of 4 mm where inter-fiber void depth was relatively large was 57%, while the inter-fiber void ratio of those regions where inter-fiber void ratio was relatively small was 40%. In addition, the inter-fiber void ratio of air-through non-woven fabric that was not processed (namely, having a tooth depth of 0 mm) was 28%.

Furthermore, the methods used to evaluate each of the evaluation parameters of Table 2 were as described below.

### [Thickness]

Thickness was measured using a thickness gauge (Peacock Corp., Model J-B, measuring surface: 50 mmφ, measuring pressure: 3 g/cm²).

### [Density]

The weight of the non-woven fabric was measured using an electronic balance. The weight was divided by the surface area of the non-woven fabric and converted to weight per square meter (m²) to calculate basis weight. Basis weight was then divided by thickness and converted to weight per cubic meter (m³) to calculate density.

### [Elasticity]

(1) The non-woven fabric was cut to a length of 100 mm in the direction of stretching and a length of 25 mm in the direction perpendicular to the direction of stretching to prepare a test piece.
(2) Clips were respectively attached to 25 mm portions on both ends of the test piece in the direction of stretching so that the distance between the clips was 50 mm (initial length: 50 mm).
(3) The test piece was arranged so as to be parallel to a test stand, one end of the test piece was anchored with a clip and a digital force gauge was connected to the other end.
(4) The digital force gauge was pulled slowly and the length of the test piece between the clips was measured when the pulling force reached 1.0 N.
(5) The length at a pulling force of 1.0 N was then divided by the initial length to determine elasticity.

### [Moisture Absorption Multiple]

After immersing a sample in physiological saline for 3 minutes, the sample was removed from the physiological saline and weighed, after which the ratio of the weight of the sample prior to immersion to the weight of the sample after immersion was taken to be the moisture absorption multiple.

### INDUSTRIAL APPLICABILITY

The absorbent article of the present invention can be preferably used in a sanitary napkin, disposable diaper and the like.

### BRIEF DESCRIPTION OF THE REFERENCE SYMBOLS

1 Absorbent article
2 Liquid-permeable top sheet
3 Liquid-impermeable back sheet
4 Superabsorbent polymer-containing layer
5 Non-woven fabric layer
6 Absorbent body
7 Regions where inter-fiber void ratio is relatively small
8 Regions where inter-fiber void ratio is relatively large
9 Wrapping sheet
51,52 Teeth
53,54 Gear rollers
55 Non-woven fabric
60 Fractured end

## Claims

1. An absorbent article having a liquid-permeable top sheet, a liquid-impermeable back sheet and an absorbent body positioned between both of these sheets, wherein the absorbent body is composed of a superabsorbent polymer-containing layer and a non-woven fabric layer, the non-woven fabric layer has regions where inter-fiber void ratio is relatively large and regions where inter-fiber void ratio is relatively small, the ends of the fibers that compose the non-woven fabric layer are more numerous in those regions where the inter-fiber void ratio is relatively large than in those regions where the inter-fiber void ratio is relatively small, and the non-woven fabric layer has expansibility.

2. The absorbent article according to claim 1, wherein elasticity of the non-woven fabric layer differs according to direction.

3. The absorbent article according to claim 1 or claim 2, wherein the non-woven fabric layer has elasticity of 1.4 times or more in at least one direction.

4. The absorbent article according to any of claims 1 to 3, wherein regions where inter-fiber void ratio is relatively large and regions where inter-fiber void ratio is relatively small are alternately formed continuously in the non-woven fabric layer, and the elasticity of the non-woven fabric layer is greater in the direction perpendicular to the direction in which those regions where inter-fiber void ratio is relatively small extend than in the direction in which those regions where inter-fiber void ratio is relatively small extend.

5. The absorbent article according to claim 4, wherein elasticity in the direction perpendicular to the direction in which those regions where inter-fiber void ratio is relatively small extend is 1.4 times or more, and elasticity in the direction in which those regions where inter-fiber void ratio is relatively small is less than 1.35 times.

6. The absorbent article according to any of claims 1 to 5, wherein the ends of the fibers are fractured ends.

7. The absorbent article according to any of claims 1 to 6, wherein the non-woven fabric layer has a density of 0.05 g/cm³ or less.

8. The absorbent article according to any of claims 1 to 7, wherein the non-woven fabric layer is composed of a non-woven fabric processed with biting gear rollers.

9. The absorbent article according to claim 8, wherein the non-woven fabric layer is composed of an air-through non-woven fabric processed with biting gear rollers having a tooth pitch of 2 mm to 8 mm and a tooth depth of 3 mm to 6 mm.

10. The absorbent article according to any of claims 1 to 9, wherein the absorbent body is composed of at least one layer of a superabsorbent polymer-containing layer and at least two layers of a non-woven fabric layer, and the superabsorbent polymer-containing layer is positioned between the non-woven fabric layers.

11. The absorbent article according to any of claims 1 to 10, wherein the superabsorbent polymer-containing layer further contains moisture absorbent fibers.

12. The absorbent article according to claim 11, wherein at least a portion of fibers that compose the non-woven fabric layer and the moisture absorbent fibers that compose the superabsorbent polymer-containing layer are bonded at the interface between the non-woven fabric layers and the superabsorbent polymer-containing layer.

13. The absorbent article according to any of claims 1 to 12, wherein the absorbent body is preferably molded by applying water or steam.

14. The absorbent article according to any of claims 1 to 13, wherein the superabsorbent polymer-containing layer is divided into a plurality of parts in a machine direction.

15. An absorbent article having a liquid-permeable top sheet, a liquid-impermeable back sheet, an absorbent body positioned between both of these sheets, and a wrapping sheet that surrounds the absorbent body, wherein the absorbent body is composed of a superabsorbent polymer-containing layer and a non-woven fabric layer, the non-woven fabric layer has regions where inter-fiber void ratio is relatively large and regions where inter-fiber void ratio is relatively small, the ends of the fibers that compose the non-woven fabric layer are more numerous in those regions where the inter-fiber void ratio is relatively large than in those regions where the inter-fiber void ratio is relatively small, the non-woven fabric layer has elasticity of 1.4 times or more in at least one direction, and the density of the non-woven fabric layer is 0.05 g/cm³ or less.
